# EUROPEAN PATENT APPLICATION

(11) **EP 3 424 995 A2**
(43) Date of publication of application: **09.01.2019**
(21) Application number: 18186672.4
(22) Date of filing: 30.05.2013
(51) Int. Cl.: C08K 9/10, B32B 38/00

(54) **A COMPOSITE MATERIAL METHOD OF PRODUCING THE SAME, AND ARTICLES MADE THEREFROM**

(30) Priority: 28.06.2012 US 201261665389 P
(62) Divisional of application: 13728619.1
(71) Applicant: Dow Global Technologies Llc, Midland, MI 48674 (US)
(72) Inventor: STUCCHI, Gloria, Midland, MI 48674 (US); KRONAWITTLEITHNER, Kurt, 8810 Horgen (CH); PRIETO, Miguel, 8805 Richterswil (CH)
(74) Representative: Beck Greener

(57) **Abstract**

A composite material, comprising: (A) a particle comprising: (i) a core comprising one or more magnetic materials and (ii) a shell comprising silicon dioxide and (B) a polymer component selected from the group consisting of polyolefin homopolymers, polyolefin interpolymers, and combinations thereof, wherein the polymer component is free of free radical initiator is provided.

## Description

### Field of Invention

The instant invention relates to a composite material, method of producing the same, articles made therefrom, and methods for making such articles.

### Background of the Invention

Thermoplastic elastomers are used in a variety of applications in the preparation of consumer products, including durable goods and consumable products. For example, thermoplastic elastomers are used in the conveyor belts used in manufacturing such goods and products as well as in certain packaging of such goods and products. Damaged conveyor belts and/or packaging can release small pieces of thermoplastic material which may contaminate the packaged goods. Such contamination may present a significant quality control problem in the food, medical, and packaging industries. Such pieces of thermoplastic material are not detectable by metal detectors which are standard installation in packaging lines.

There are a number of metal detectable conveyor belts using a metal oxide as the signal source. However, such metals are not detectable in all applications. For example, iron oxide is detectable in wet product applications but not in dry applications. Conductive inclusions (such as carbon) tend not to be detectable in wet product applications.

Therefore, there remains a need for metal detectable thermoplastic elastomer compositions useful in both wet and dry applications.

### Summary of the Invention

The instant invention is a composite material, method of producing the same, articles made therefrom, and methods for making such articles.

In one embodiment, the instant invention provides a composite material, comprising: (A) a particle comprising: (i) a core comprising one or more magnetic materials and (ii) a shell comprising silicon dioxide and (B) a polymer component selected from the group consisting of polyolefin homopolymers, polyolefin interpolymers, and combinations thereof, wherein the polymer component is free of free radical initiator.

### Detailed Description of the Invention

The instant invention is a composite material, method of producing the same, articles made therefrom, and methods for making such articles.

The composite material according to the present invention comprises: (A) a particle comprising: (i) a core comprising one or more magnetic materials and (ii) a shell comprising silicon dioxide and (B) a polymer component selected from the group consisting of polyolefin homopolymers, polyolefin interpolymers, or a combinations thereof, wherein the polymer component is free of free radical initiator.

In an alternative embodiment, the instant invention further provides a method for producing a composite material comprising the steps of: providing particles which comprise: (i) a core comprising one or more magnetic materials and (ii) a shell comprising silicon dioxide; providing a polymer component selected from the group consisting of polyolefin homopolymers, polyolefin interpolymers, or a combinations thereof, wherein the polymer component is free of free radical initiator, and forming a mixture of the particles and the polymer component.

In an alternative embodiment, the instant invention further provides an article selected from the group consisting of laminates, sheets, and films, wherein the article comprises the composite material.

In an alternative embodiment, the instant invention provides a composite material, method of producing the same, articles made therefrom, and method of making such articles, in accordance with any of the preceding embodiments, except that the article has a thickness in the range of from 0.25 to 4 mm. All individual values and subranges from 0.25 to 4 mm are included herein and disclosed herein; for example, the article thickness can be from a lower limit of 0.25, 0.5, 1, 1.5, 2, 2.25, 2.75, 3, or 3.75 mm to an upper limit of 0.75, 1.25, 1.75, 2.5, 3, 3.5 or 4 mm For example, the article thickness may be in the range of from0.25 to 4 mm, or in the alternative, the article thickness may be in the range of from 0.5 to 3 mm, or in the alternative, , or in the alternative, the article thickness may be in the range of from 0.5 to 1.5 mm, or in the alternative, the article thickness may be in the range of from 0.75 to 2 mm.

In an alternative embodiment, the invention further provides a conveyor belt which comprises an article in accordance with the preceding embodiment.

In yet another alternative embodiment, the instant invention further provides a method for making an article comprising forming an article from one or more composite materials, wherein the step of forming is selected from the group of extruding, calendaring, and molding.

For the purposes of the invention, composite material is a physical mixture of the components.

In an alternative embodiment, the instant invention provides a composite material, method of producing the same, articles made therefrom, and method of making such articles, in accordance with any of the preceding embodiments, except that a distribution of the components (A) and (B) is substantially homogeneous.

In an alternative embodiment, the instant invention provides a composite material, method of producing the same, articles made therefrom, and method of making such articles, in accordance with any of the preceding embodiments, except that the polyolefin interpolymer is a polyolefin copolymer.

In an alternative embodiment, the instant invention provides a composition, method of producing the same, articles made therefrom, and method of making such articles, in accordance with any of the preceding embodiments, except that the polymer component is selected from the group consisting of polyethylene homopolymers, polyethylene/α-olefin copolymers, polypropylene homopolymers, polypropylene/α-olefin copolymers, and combinations thereof.

In an alternative embodiment, the instant invention provides a composite material, method of producing the same, articles made therefrom, and method of making such articles, in accordance with any of the preceding embodiments, except that the particle (A) is present in an amount from 2 to 50% by weight based on the total weight of the composite material.

All individual values and subranges from 2 to 50 wt% are included herein and disclosed herein; for example, the amount of particles (A) can be from a lower limit of 2, 12, 18, 22, 28, 32, 38, 42, or 48 wt% to an upper limit of 3, 13, 20, 29, 33, 38, 43, 47 or 50 wt%. For example, the amount of particles (A) may be in the range of from 2 to 50 wt%, or in the alternative, amount of particles (A) may be in the range of from 2.5 to 5 wt%, or in the alternative, amount of particles (A) may be in the range of from 5 to 10 wt%, or in the alternative, amount of particles (A) may be in the range of from 7.5 to 15 wt%..

In an alternative embodiment, the instant invention provides a composition, method of producing the same, articles made therefrom, and method of making such articles, in accordance with any of the preceding embodiments, except that the polymer component is present in an amount from 50 to 98% by weight based on the total weight of the composite material.

All individual values and subranges from50 to 98 wt% are included herein and disclosed herein; for example, the amount of the polymer component in the composite material can be from a lower limit of 50, 60, 72, 80, 91 or 97 wt% to an upper limit of 51, 63, 75, 83, 92, or 98 wt%. For example, the amount of the polymer component may be in the range of from 50 to 98 wt%, or in the alternative, the amount of the polymer component may be in the range of from 90 to 98 wt%, or in the alternative, the amount of the polymer component may be in the range of from 95 to 97.5 wt%, or in the alternative, the amount of the polymer component may be in the range of from 85 to 92.5 wt%.

For the purposes of the invention, particles with a core-shell structure are particles which are: (i) isolated individual particles surrounded by a shell, (ii) aggregates of accreted cores, where the aggregates have been surrounded by a shell and/or (iii) aggregates accreted by way of the shells. Aggregates are individual particles firmly accreted, for example by way of sinter necks.

In an alternative embodiment, the instant invention provides a composite material, method of producing the same, articles made therefrom, and method of making such articles, in accordance with any of the preceding embodiments, except that the shell of the particles which have core-shell structure and which are present in the composite material according to the invention can be one or more shells surrounding the core.

In an alternative embodiment, the instant invention provides a composite material, method of producing the same, articles made therefrom, and method of making such articles, in accordance with any of the preceding embodiments, except that the one or more shells comprises silicon dioxide.

In an alternative embodiment, the instant invention provides a composite material, method of producing the same, articles made therefrom, and method of making such articles, in accordance with any of the preceding embodiments, except that an outermost shell is perforation-free and consists essentially of silicon dioxide.

In an alternative embodiment, the instant invention provides a composite material, method of producing the same, articles made therefrom, and method of making such articles, in accordance with any of the preceding embodiments, except that the an outermost shell completely encloses or surrounds the core.

In an alternative embodiment, the instant invention provides a composite material, method of producing the same, articles made therefrom, and method of making such articles, in accordance with any of the preceding embodiments, except that the shell comprises more than one shell and the inner shells are not perforation-free.

Such inner shells may comprise compounds composed of the elements involved in the shell material and the elements involved in the core material. By way of example, this can be iron silicate if the core comprises iron or iron compounds.

In an alternative embodiment, the instant invention provides a composite material, method of producing the same, articles made therefrom, and method of making such articles, in accordance with any of the preceding embodiments, except that the thickness of the shell is in the nanometer range.

In an alternative embodiment, the instant invention provides a composite material, method of producing the same, articles made therefrom, and method of making such articles, in accordance with any of the preceding embodiments, except that the thickness of the shell is from 2 to 500 nm. All individual values and subranges from 2 to 500 nm are included herein and disclosed herein; for example, the thickness of the shell can be from a lower limit of 2, 12, 18, 24, 30, 38, 44, or 49 nm to an upper limit of 3, 9, 15, 20, 29, 37, 45 or 50 nm. For example, the shell thickness may be in the range of from 2 to 50 nm, or in the alternative, the shell thickness may be in the range of from 5 to 30 nm, or in the alternative, the shell thickness may be in the range of from 20 to 40 nm, or in the alternative, the shell thickness may be in the range of from 40 to 50 nm.

In an alternative embodiment, the instant invention provides a composite material, method of producing the same, articles made therefrom, and method of making such articles, in accordance with any of the preceding embodiments, except that the shell is substantially pore-free. In an alternative embodiment, the instant invention provides a composite material, method of producing the same, articles made therefrom, and method of making such articles, in accordance with any of the preceding embodiments, except that the thickness of the shell has free hydroxy groups on the surface.

Magnetic materials useful in embodiments of the invention are paramagnetic, ferromagnetic, ferrimagnetic, or superparamagnetic materials, or a mixture of these. In an alternative embodiment, the instant invention provides a composite material, method of producing the same, articles made therefrom, and method of making such articles, in accordance with any of the preceding embodiments, except that the magnetic material is a selected from the group consisting of superparamagnetic material and materials which have only slight remnant magnetization.

In an alternative embodiment, the instant invention provides a composite material, method of producing the same, articles made therefrom, and method of making such articles, in accordance with any of the preceding embodiments, except that the particles (A) comprise superparamagnetic material and further exhibit hysteresis.

In an alternative embodiment, the instant invention provides a composite material, method of producing the same, articles made therefrom, and method of making such articles, in accordance with any of the preceding embodiments, except that the core material is selected from the group consisting of Fe, Co and Ni; oxides of Fe, Co and/or Ni, such as Fe₃O₄ and gamma-Fe₂O₃; spinel-type ferromagnetic materials such as MgFe₂O₄, MnFe₂O₄ and CoFe₂O₄; alloys, such as CoPt₃ and FePt; and combinations thereof.

In an alternative embodiment, the instant invention provides a composite material, method of producing the same, articles made therefrom, and method of making such articles, in accordance with any of the preceding embodiments, except that the core material comprises one or more iron oxides selected from the group consisting of haematite, magnetite and maghemite, or a mixture of two or three of these iron oxides.

In an alternative embodiment, the instant invention provides a composite material, method of producing the same, articles made therefrom, and method of making such articles, in accordance with any of the preceding embodiments, except that the core material consists essentially of one or more iron oxides selected from the group consisting of haematite, magnetite and maghemite, or a mixture of two or three of these iron oxides.

The proportions of core material and of shell material within the core/shell structure can vary within wide limits as a function of core material, of the thickness of the shell, and of the structure of the particles, isolated or aggregated. The proportions of the core material and of the shell material are generally in each case from 10 to 90% by weight.

All individual values and subranges from 10 to 90 wt% are included herein and disclosed herein; for example, the amount of core in the core/shell structure can be from a lower limit of 10, 20, 30, 40, 50, 60, 70, 80, or 89 wt% to an upper limit of 15, 25, 35, 45, 55, 65, 75, 85 or 90 wt%. For example, the amount of the core in the core/shell structure may be in the range of from 10 to 90 wt%, or in the alternative, the amount of the core in the core/shell structure may be in the range of from 50 to 90 wt%, or in the alternative, the amount of the core in the core/shell structure may be in the range of from 50 to 80 wt%, or in the alternative, the amount of the core in the core/shell structure may be in the range of from 75 to 85 wt%.

Likewise, all individual values and subranges from 10 to 90 wt% with respect to the amount of shell in the core/shell structure are included herein and disclosed herein; for example, the amount of shell in the core/shell structure can be from a lower limit of 10, 20, 30, 40, 50, 60, 70, 80, or 89 wt% to an upper limit of 15, 25, 35, 45, 55, 65, 75, 85 or 90 wt%. For example, the amount of the shell in the core/shell structure may be in the range of from 10 to 90 wt%, or in the alternative, the amount of the shell in the core/shell structure may be in the range of from 10 to 50 wt%, or in the alternative, the amount of the shell in the core/shell structure may be in the range of from 30 to 50 wt%, or in the alternative, the amount of the core in the core/shell structure may be in the range of from 15 to 25 wt%.

In an alternative embodiment, the instant invention provides a composite material, method of producing the same, articles made therefrom, and method of making such articles, in accordance with any of the preceding embodiments, except that the BET surface area of the core/shell particles can be from 5 to 500 m²/g. All individual values and subranges from 5 to 500 m²/g are included herein and disclosed herein; for example, the BET surface area of the core/shell particles can be from a lower limit of 5, 50, 100, 150, 200, 250, 300, 350, 400 or 450 m²/g to an upper limit of 10, 20, 110, 160, 210, 260, 310, 360, 410, 460 or 500 m²/g. For example, the BET surface area of the core/shell particles may be in the range of from 5 to 500 m²/g, or in the alternative, the BET surface area of the core/shell particles may be in the range of from 30 to 300 m²/g, or in the alternative, the BET surface area of the core/shell particles may be in the range of from 40 to 150 m²/g, or in the alternative, the BET surface area of the core/shell particles may be in the range of from 50 to 100 m²/g.

In an alternative embodiment, the instant invention provides a composite material, method of producing the same, articles made therefrom, and method of making such articles, in accordance with any of the preceding embodiments, except that the average diameter of the particles is from 5 to 100 nm. All individual values and subranges from 5 to 100 nm are included herein and disclosed herein; for example, the average diameter of the particles can be from a lower limit of 5, 15, 25, 35, 45, 55, 65, 75, 85 or 95 nm to an upper limit of 10, 20, 30, 40, 50, 60, 70, 80, 90 or 100 nm. For example, the average diameter of the particles may be in the range of from 5 to 100 nm, or in the alternative, average diameter of the particles may be in the range of from 30 to 80 nm, or in the alternative, average diameter of the particles may be in the range of from 50 to 70 nm, or in the alternative, average diameter of the particles may be in the range of from 55 to 65 nm.

In an alternative embodiment, the instant invention provides a composite material, method of producing the same, articles made therefrom, and method of making such articles, in accordance with any of the preceding embodiments, except that the 90% spread of the proportional distribution of the particles is from 5 to 60 nm. All individual values and subranges from 5 to 60 nm are included herein and disclosed herein; for example, the 90% spread of the proportional distribution of the particles can be from a lower limit of 5, 15, 25, 35, 45, or 55 nm to an upper limit of 10, 20, 30, 40, 50 or 60 nm. For example, the 90% spread of the proportional distribution of the particles may be in the range of from5 to 60 nm, or in the alternative, the 90% spread of the proportional distribution of the particles may be in the range of from 15 to 50 nm, or in the alternative, the 90% spread of the proportional distribution of the particles may be in the range of from 5 to 40 nm.

In an alternative embodiment, the instant invention provides a composite material, method of producing the same, articles made therefrom, and method of making such articles, in accordance with any of the preceding embodiments, except that the particles having core/shell structure are surface-modified. For the purposes of the invention, surface-modified means that at least a portion of the hydroxy groups located on the surface of the powder have reacted with a surface modifier to form a chemical bond. The chemical bond is preferably a covalent bond, ionic bond or coordinative bond with formation of a complex between the surface modifier and the particle. A coordinative bond means formation of a complex.

The surface modifier can preferably be surface modifiers which have, as functional group, a carboxylic acid group, an acyl chloride group, an ester group, a nitrile group, an isonitrile group, a hydroxy group, a thiol group, an epoxy group, an anhydride group, an amide group, an amino group, or a silanol group.

In an alternative embodiment, the instant invention provides a composite material, method of producing the same, articles made therefrom, and method of making such articles, in accordance with any of the preceding embodiments, except that the surface modifiers are silanes which have at least one non-hydrolysable group or one hydroxy group, in particular hydrolysable organosilanes which also have at least one non-hydrolysable moiety.

Examples are silanes of the general formula RₐSiX₄₋ₐ, in which the moieties R are identical or different and are non-hydrolysable groups, the moieties X are identical or different and are hydrolysable groups or hydroxy groups, and a is the value 1, 2 or 3. The value of a is preferably 1. Examples of the hydrolysable groups X in the general formula, where these can be identical or differ from one another, are hydrogen or halogen, F, Cl, Br or I; alkoxy, in particular C₁-C₆-alkoxy, such as methoxy, ethoxy, n-propoxy, isopropoxy and butoxy; aryloxy, in particular C₆-C₁₀-aryloxy, such as phenoxy; acyloxy, in particular C₁-C₆-acyloxy, such as acetoxy or propionyloxy; alkylcarbonyl, in particular C₂-C₇-alkylcarbonyl, such as acetyl; amino, in particular monoalkylamino or dialkylamino.

In a particular embodiment, the hydrolysable moieties are halogen, alkoxy groups and acyloxy groups. In another embodiment, the hydrolysable moieties are C₁-C₄-alkoxy groups, in particular methoxy and ethoxy.

The non-hydrolysable moieties R which can be identical or differ from one another can be non-hydrolysable moieties R having or not having a functional group. By way of example, the non-hydrolysable moiety R not having a functional group can be alkyl, in particular C₁-C₈-alkyl, such as methyl, ethyl, n-propyl, isopropyl, n-butyl, sec-butyl and tert-butyl, pentyl, hexyl, octyl or cyclohexyl; alkenyl, in particular C₂-C₆-alkenyl, such as vinyl, 1-propenyl, 2-propenyl and butenyl; alkynyl, in particular C₂-C₆-alkynyl, such as acetylenyl and propargyl; aryl, in particular C₆-C₁₀-aryl, such as phenyl and naphthyl, and also corresponding alkaryl moieties, such as tolyl, benzyl and phenethyl.

In yet other embodiments, the surface modifiers are selected from the group consisting of CH SiCl₃, CH₃Si(OC2H₅)₃, CH₃Si(OCH₃)₃, C₂H₅SiCl₃, C₂H₅Si(OC₂H₅)₃, C₂H₅Si(OCH₃)₃, C₃H₇Si(OC₂H₅)₃, (C₂H₅O)₃SiC₃H₆Cl, (CH₃)₂SiCl₂, (CH₃)₂Si(OC₂H₅)₂, (CH₃)₂Si(OH)₂, C₆H₅Si(OCH₃)₃, C₆H₅Si(OC₂H₅)₃, C₆H₅CH₂CH₂Si(OCH₃)₃, (C₆H₅)₂SiCl₂, (C₆H₅)₂Si(OC₂H₅)₂, (iso-C₃H₇)₃SiOH, CH₂=CHSi(OOCCH₃)₃, CH₂=CHSiCl₃, CH₂=CH-Si(OC₂H₅)₃, CH₂=CHSi(OC₂H₅)₃, CH₂=CH--Si(OC₂H₄OCH₃)₃, CH₂=CH--CH₂-Si(OC₂H₅)₃, CH₂=CH--CH₂²-Si(OC₂H₅)₃, CH₂=CH₂--Si(OOOC₂H₃)₃, n-C₆H₁₃-CH₂-CH₂-Si(OC₂H₅)₃, n-C₈H₁₇--CH₂CH₂-Si(OC₂H₅)₃, .gamma.-glycidyloxypropyltrimethoxysilane, .gamma.-glycidyloxypropyltriethoxysilane, 3-isocyanatopropyl-triethoxysilane, 3-isocyanatopropyldimethylchlorosilane, 3-aminopropyltrimethoxysilane, 3-aminopropyltriethoxysilane, N-(2-aminoethyl)-3-aminopropyltrimethoxysilane, N-[N'-(2'-aminoethyl)-2-aminoethyl]-3-aminopropyltrimethoxysilane, hydroxymethyltriethoxysilane, 2-[methoxy(polyethyleneoxy)propyl]trimethoxysilane, bis(hydroxyethyl)-3-aminopropyltriethoxysilane, N-hydroxyethyl-N-methylaminopropyltriethoxysilane, 3-(meth)acryloxypropyltriethoxysilane and 3-(meth)acryloxypropyltrimethoxysilane.

In yet another embodiment, the surface modifiers are selected from the group consisting of octyltrimethoxysilane, octyltriethoxysilane, hexamethyldisilazane, 3-methacryloxypropyltrimethoxysilane, 3-methacryloxypropyltriethoxysilane, dimethylpolysiloxane, glycidyloxypropyltrimethoxysilane, glycidyloxypropyl-triethoxysilane, nonafluorohexyltrimethoxysilane, tridecaflourooctyltrimethoxysilane, tridecaflourooctyltriethoxysilane, aminopropyltriethoxysilane, and oligomeric, short-chain, alkyl-functionalized silanes. The following can be very particularly preferred: octyltrimethoxysilane, octyltriethoxysilane, dimethylpoly-siloxanes and oligomeric, short-chain, alkyl-functionalized silanes.

In an alternative embodiment, the instant invention provides a composite material, method of producing the same, articles made therefrom, and method of making such articles, in accordance with any of the preceding embodiments, except that the particles with core/shell structure have a carbon content from 0.1 to 10% by weight, as a function of the nature of the surface-modifying reagent and the amount thereof. All individual values and subranges from 0.1 to 10% by weight are included herein and disclosed herein; for example, the carbon content of the core/shell structure can be from a lower limit of 0.1, 2, 4, 6, or 8 wt% to an upper limit of 0.2, 3, 5, 7, 9 or 10 wt%. For example, the carbon content of the core/shell structure may be in the range of from 0.1 to 10 wt%, or in the alternative, the carbon content of the core/shell structure may be in the range of from 1 to 6 wt%.

The polymer component (B) is selected from the group consisting of polyolefin homopolymers, polyolefin interpolymers and combinations thereof.

As used herein, the term "ethylene-based polymer" refers to a polymer that is formed from more than 50 mole percent polymerized ethylene monomer (based on the total amount of polymerizable monomers), and, optionally, one or more comonomers.

As used herein, the term "propylene-based polymer" refers to a polymer that is formed from more than 50 mole percent polymerized propylene monomer (based on the total amount of polymerizable monomers), and, optionally, one or more comonomers.

As used herein, the term "ethylene/α-olefin interpolymer" refers to an interpolymer that is formed from more than 50 mole percent polymerized ethylene monomer (based on the total amount of polymerizable monomers), and at least one α-olefin comonomer.

The term "homopolymer" is a polymer that is formed from only a single type of monomer, such as ethylene.

The term "interpolymer" refers to polymers prepared by the copolymerization of at least two different types of monomers. The term interpolymer includes copolymers, usually employed to refer to polymers prepared from two different monomers, and polymers prepared from more than two different types of monomers, such as terpolymers.

In one embodiment, the polymer component (B) is selected from the group consisting of polyethylene homopolymers, polyethylene-based interpolymers, polypropylene homopolymers, polypropylene/α-olefin copolymers and combinations thereof. Exemplary polymer components include homopolymers and copolymers (including elastomers) of an α-olefin such as ethylene, propylene, 1-butene, 3-methyl-1-butene, 4-methyl-1-pentene, 3-methyl-1-pentene, 1-heptene, 1-hexene, 1-octene, 1-decene, and 1-dodecene, as typically represented by polyethylene, polypropylene, poly-1-butene, poly-3-methyl-1-butene, poly-3-methyl-1-pentene, poly-4-methyl-1-pentene, ethylene-propylene copolymer, ethylene-1-butene copolymer, and propylene-1-butene copolymer; copolymers (including elastomers) of an α-olefin with a conjugated or non-conjugated diene, as typically represented by ethylene-butadiene copolymer and ethylene-ethylidene norbornene copolymer; and polyolefins (including elastomers) such as copolymers of two or more α-olefins with a conjugated or non-conjugated diene, as typically represented by ethylene-propylene-butadiene copolymer, ethylene-propylene-dicyclopentadiene copolymer, ethylene-propylene-1,5-hexadiene copolymer, and ethylene-propylene-ethylidene norbornene copolymer; ethylene-vinyl compound copolymers such as ethylene-vinyl acetate copolymer, ethylene-vinyl alcohol copolymer, ethylene-vinyl chloride copolymer, ethylene acrylic acid or ethylene-(meth)acrylic acid copolymers, and ethylene-(meth)acrylate copolymer; styrenic copolymers (including elastomers) such as polystyrene, ABS, acrylonitrile-styrene copolymer,α-methylstyrene-styrene copolymer, styrene vinyl alcohol, styrene acrylates such as styrene methylacrylate, styrene butyl acrylate, styrene butyl methacrylate, and styrene butadienes and crosslinked styrene polymers; and styrene block copolymers (including elastomers) such as styrene-butadiene copolymer and hydrate thereof, and styrene-isoprene-styrene tri-block copolymer; polyvinyl compounds such as polyvinyl chloride, polyvinylidene chloride, vinyl chloride-vinylidene chloride copolymer, polymethyl acrylate, and polymethyl methacrylate; polyamides such as nylon 6, nylon 6,6, and nylon 12; thermoplastic polyesters such as polyethylene terephthalate and polybutylene terephthalate; polycarbonate, polyphenylene oxide, and the like; and glassy hydrocarbon-based resins, including poly-dicyclopentadiene polymers and related polymers (copolymers, terpolymers); saturated mono-olefins such as vinyl acetate, vinyl propionate and vinyl butyrate and the like; vinyl esters such as esters of monocarboxylic acids, including methyl acrylate, ethyl acrylate, n-butylacrylate, isobutyl acrylate, dodecyl acrylate, n-octyl acrylate, phenyl acrylate, methyl methacrylate, ethyl methacrylate, and butyl methacrylate and the like; acrylonitrile, methacrylonitrile, acrylamide, mixtures thereof; resins produced by ring opening metathesis and cross metathesis polymerization and the like. These resins may be used either alone or in combinations of two or more.

In one particular embodiment, the thermoplastic resin may comprise an α-olefin interpolymer of ethylene with a comonomer comprising an alkene, such as 1-octene.

Ethylene α-olefin multi-block interpolymers used in embodiments disclosed herein may be interpolymers of ethylene with at least one C₃-C₂₀ α-olefin. The interpolymers may further comprise C₄-C₁₈ diolefin and/or alkenylbenzene. Suitable unsaturated comonomers useful for polymerizing with ethylene include, for example, ethylenically unsaturated monomers, conjugated or non-conjugated dienes, polyenes, alkenylbenzenes, etc. Examples of such comonomers include C₃-C₂₀ α-olefins such as propylene, isobutylene, 1-butene, 1-hexene, 1-pentene, 4-methyl-1-pentene, 1-heptene, 1-octene, 1-nonene, 1-decene, and the like. In certain embodiments, the α-olefins may be 1-Butene or 1-octene. Other suitable monomers include styrene, halo- or alkyl-substituted styrenes, vinylbenzocyclobutane, 1,4-hexadiene, 1,7-octadiene, and naphthenics (such as cyclopentene, cyclohexene, and cyclooctene, for example).

Embodiments disclosed herein may also include a polymeric component that may include at least one multi-block olefin interpolymer. Suitable multi-block olefin interpolymers may include those described in U.S. Provisional Patent Application No. 60/818,911, for example. The term "multi-block copolymer" or refers to a polymer comprising two or more chemically distinct regions or segments (referred to as "blocks") preferably joined in a linear manner, that is, a polymer comprising chemically differentiated units which are joined end-to-end with respect to polymerized ethylenic functionality, rather than in pendent or grafted fashion.

The multi-block interpolymers disclosed herein may be differentiated from conventional, random copolymers, physical blends of polymers, and block copolymers prepared via sequential monomer addition, fluxional catalysts, and anionic or cationic living polymerization techniques. In particular, compared to a random copolymer of the same monomers and monomer content at equivalent crystallinity or modulus, the interpolymers have better (higher) heat resistance as measured by melting point, higher TMA penetration temperature, higher high-temperature tensile strength, and/or higher high-temperature torsion storage modulus as determined by dynamic mechanical analysis.

Other olefin interpolymers include polymers comprising monovinylidene aromatic monomers including styrene, o-methyl styrene, p-methyl styrene, t-butylstyrene, and the like. In particular, interpolymers comprising ethylene and styrene may be used. In other embodiments, copolymers comprising ethylene, styrene and a C₃-C₂₀ α-olefin, optionally comprising a C₄-C₂₀ diene, may be used.

Suitable non-conjugated diene monomers may include straight chain, branched chain or cyclic hydrocarbon diene having from 6 to 15 carbon atoms. Examples of suitable non-conjugated dienes include, but are not limited to, straight chain acyclic dienes, such as 1,4-hexadiene, 1,6-octadiene, 1,7-octadiene, 1,9-decadiene, branched chain acyclic dienes, such as 5-methyl-1,4-hexadiene; 3,7-dimethyl-1,6-octadiene; 3,7-dimethyl-1,7-octadiene and mixed isomers of dihydromyricene and dihydroocinene, single ring alicyclic dienes, such as 1,3-cyclopentadiene; 1,4-cyclohexadiene; 1,5-cyclooctadiene and 1,5-cyclododecadiene, and multi-ring alicyclic fused and bridged ring dienes, such as tetrahydroindene, methyl tetrahydroindene, dicyclopentadiene, bicyclo-(2,2,1)-hepta-2,5-diene; alkenyl, alkylidene, cycloalkenyl and cycloalkylidene norbornenes, such as 5-methylene-2-norbomene (MNB); 5-propenyl-2-norbornene, 5-isopropylidene-2-norbornene, 5-(4-cyclopentenyl)-2-norbornene, 5-cyclohexylidene-2-norbornene, 5-vinyl-2-norbornene, and norbomadiene. Of the dienes typically used to prepare EPDMs, the particularly preferred dienes are 1,4-hexadiene (HD), 5-ethylidene-2-norbornene (ENB), 5-vinylidene-2-norbornene (VNB), 5-methylene-2-norbornene (MNB), and dicyclopentadiene (DCPD).

One class of desirable polymers that may be used in accordance with embodiments disclosed herein includes elastomeric interpolymers of ethylene, a C₃-C₂₀ α-olefin, especially propylene, and optionally one or more diene monomers. Preferred α-olefins for use in this embodiment are designated by the formula CH₂=CHR*, where R* is a linear or branched alkyl group of from 1 to 12 carbon atoms. Examples of suitable α-olefins include, but are not limited to, propylene, isobutylene, 1-butene, 1-pentene, 1-hexene, 4-methyl-1-pentene, and 1-octene. A particularly preferred α-olefin is propylene. The propylene based polymers are generally referred to in the art as EP or EPDM polymers. Suitable dienes for use in preparing such polymers, especially multi-block EPDM type polymers include conjugated or non-conjugated, straight or branched chain-, cyclic- or polycyclicdienes comprising from 4 to 20 carbons. Preferred dienes include 1,4-pentadiene, 1,4-hexadiene, 5-ethylidene-2-norbornene, dicyclopentadiene, cyclohexadiene, and 5-butylidene-2-norbornene. A particularly preferred diene is 5-ethylidene-2-norbornene.

In specific embodiments, polyolefins such as polypropylene, polyethylene, copolymers thereof, and blends thereof, as well as ethylene-propylene-diene terpolymers, may be used. In some embodiments, preferred olefinic polymers include homogeneous polymers, as described in U.S. Pat. No. 3,645,992 issued to Elston; high density polyethylene (HDPE), as described in U.S. Pat. No. 4,076,698 issued to Anderson; heterogeneously branched linear low density polyethylene (LLDPE); heterogeneously branched ultra low linear density polyethylene (ULDPE); homogeneously branched, linear ethylene/α-olefin copolymers; homogeneously branched, substantially linear ethylene/α-olefin polymers, which can be prepared, for example, by processes disclosed in U.S. Pat. Nos. 5,272,236 and 5,278,272, the disclosures of which are incorporated herein by reference; and high pressure, free radical polymerized ethylene polymers and copolymers such as low density polyethylene (LDPE) or ethylene vinyl acetate polymers (EVA).

Polymer compositions, and blends thereof, described in U.S. Pat. Nos. 6,566,446, 6,538,070, 6,448,341, 6,316,549, 6,111,023, 5,869,575, 5,844,045, or 5,677,383, each of which is incorporated herein by reference in its entirety, may also be suitable in some embodiments. In some embodiments, the blends may include two different Ziegler-Natta polymers. In other embodiments, the blends may include blends of a Ziegler-Natta polymer and a metallocene polymer. In still other embodiments, the polymer used herein may be a blend of two different metallocene polymers. In other embodiments, single site catalyst polymers may be used.

In some embodiments, the polymer is a propylene-based copolymer or interpolymer. In some particular embodiments, the propylene/ethylene copolymer or interpolymer is characterized as having substantially isotactic propylene sequences. The term "substantially isotactic propylene sequences" and similar terms mean that the sequences have an isotactic triad (mm) measured by .sup.13C NMR of greater than about 0.85 in one embodiment; greater than about 0.90 in another embodiment; greater than about 0.92 in another embodiment; and greater than about 0.93 in yet another embodiment. Isotactic triads are well-known in the art and are described in, for example, U.S. Pat. No. 5,504,172 and WO 00/01745, which refer to the isotactic sequence in terms of a triad unit in the copolymer molecular chain determined by ¹³C NMR spectra.

Suitable substantially linear polymers useful in one embodiment of the invention include ENGAGE polymers and AFFINITY polymers (both available from The Dow Chemical Company).

Propylene-based polymers useful in certain embodiments of the invention include propylene homopolymer (hPP), and propylene interpolymers, including for example, random propylene interpolymer (rPP). Suitable propylene-based interpolymers useful in one embodiment of the invention include VERSIFY polymers (available from The Dow Chemical Company) and VISTAMAXX polymers (available from ExxonMobil Chemical Co.).

In an alternative embodiment, the instant invention provides a composite material, method of producing the same, articles made therefrom, and method of making such articles, in accordance with any of the preceding embodiments, except that the polymer component does not include any copolymer units derived from a copolymer selected from the group consisting of ethylene-vinyl acetate (EVA), ethylene-butyl acrylate (EBA), ethylene-ethyl acrylate (EEA) and/or ethylene-methyl acrylate (EMA).

In an alternative embodiment, the instant invention provides a composite material, comprising: (A) a particle consisting essentially of: (i) a core comprising one or more magnetic materials and (ii) a shell comprising silicon dioxide and (B) a polymer component selected from the group consisting of polyolefin homoploymers, polyolefin interpolymers, and combinations thereof, wherein the polymer component is free of free radical initiator is provided.

In an alternative embodiment, the instant invention provides a composite material, consisting essentially of: (A) a particle comprising: (i) a core comprising one or more magnetic materials and (ii) a shell comprising silicon dioxide and (B) a polymer component selected from the group consisting of polyolefin homoploymers, polyolefin interpolymers, and combinations thereof, wherein the polymer component is free of free radical initiator is provided.

In an alternative embodiment, the instant invention provides a composite material, consisting essentially of: (A) a particle consisting essentially of: (i) a core comprising one or more magnetic materials and (ii) a shell comprising silicon dioxide and (B) a polymer component selected from the group consisting of polyolefin homoploymers, polyolefin interpolymers, and combinations thereof, wherein the polymer component is free of free radical initiator is provided.
In an alternative embodiment, the instant invention provides a composite material, consisting essentially of: (A) a particle consisting essentially of: (i) a core consisting essentially of one or more magnetic materials and (ii) a shell consisting essentially of silicon dioxide and (B) a polymer component selected from the group consisting of polyolefin homoploymers, polyolefin interpolymers, and combinations thereof, wherein the polymer component is free of free radical initiator is provided.

A conveyor belt may comprise one or more layers.

As used herein in the context of a conveyor belt, the term "external layer" means the layer of material in contact with the items or goods being conveyed on the conveyor belt.

As used herein in the context of a conveyor belt, the term "internal layer" means one or more layers of a conveyor belt which are not in direct contact with the items or goods being conveyed on the conveyor belt.

Magnetic particles, such as the core/shell particles used in any of the preceding embodiments, may upon exposure to electromagnetic radiation heat up. Such heating could further result in a temperature increase in the surrounding polymer component which in turn could cause a softening of the polymer component.

In another embodiment, the invention provides a method for self - healing or self - welding a conveyor belt comprising one or more layer comprising the inventive composite material comprising the steps of: providing a conveyor belt which comprises an article which comprises the composite material of any of the foregoing embodiments as an external layer, as an internal layer(s) or as a combination of internal layer(s) and external layer; and applying radiation to the conveyor belt.

In an alternative embodiment, the radiation is selected from the group consisting of electromagnetic and microwave radiation.

In another alternative embodiment, the radiation is applied to selectively cause a rise in temperature of the core/shell particles.

In yet another embodiment, the method of self- healing or self - welding a conveyor further comprises applying heat to a damaged portion of the conveyor belt.

### Examples

The following examples illustrate the present invention but are not intended to limit the scope of the invention.

Six samples are formed having varying amounts of core/shell particles dispersed in thermoplastic elastomer components. Table 1 below shows the compositions of Inventive Examples 1-5.

**Table 1**

| **Sample** | **AMPLIFY EA 103 (wt%)** | **PRIMACOR 5890 (wt%)** | **MAGSILICA (wt%)** |
|---|---|---|---|
| Inventive Ex. 1 | 85 | 10 | 5 |
| Inventive Ex. 2 | 75 | 10 | 15 |
| Inventive Ex. 3 | 70 | 20 | 10 |
| Inventive Ex. 4 | 55 | 20 | 20 |
| Inventive Ex. 5 | 80 | 0 | 20 |

Inventive Example 6 contains 47 wt% AFFINITY GA 1950, 3 wt% STRUKTOL WB42, and 50 wt% MAGSILICA 50-85. AMPLIFY EA 103, an ethylene-ethyl acrylate (EEA) copolymer having a density of 0.930 g/cm³ (measured according to ASTM D792) and an I₂ of 21 g/10min (measured according to ASTM D1238 at 190 °C and 2.16 kg), is commercially available from The Dow Chemical Company. PRIMACOR 5890, an ethylene-acrylic acid (AA) copolymer, 20 wt % AA, is commercially available from The Dow Chemical Company. MAGSILICA, particles of iron oxide particles having a size between 5 and 30 nm embedded in an amorphous silica matrix, is commercially available from Evonik Industries. STRUKTOL WB42 is synergistic blend of fatty acid derivatives with selected polarities which is commercially available from the Struktol Company (Stow, Ohio, USA). The samples are prepared in a Compounding-Haake Polylab Twinscrew extruder. Pellets are made using a pelletizer at the nozzle of the extruder. Plaques (9 cm x 5 cm x 4 mm thickness) are made using compression molding.

### Test Methods

The example composite materials are tested using a Goring Kerr DSP2 metal detector having a search head aperture 195 mm x 95 mm (width X height). The detectability reference for dry products is 120 signal with a 0.8 mm diameter ferrous sphere and the detectability reference for wet products is 120 signal with a 1.3 mm diameter metallic (non-ferrous) sphere. As can be seen in Table 2 below, pellets (diameter = 2-2.5 mm and length 2.5-3 mm) with core/shell particles present in amounts from 5 to 50 wt% based on the total weight of the composite material, are detectable under both dry and wet conditions.

The present invention may be embodied in other forms without departing from the spirit and the essential attributes thereof, and, accordingly, reference should be made to the appended claims, rather than to the foregoing specification, as indicating the scope of the invention.

**Table 2**

| **Sample** | **Ref. = 0.8** | | | **Ref. = 1.3** | | |
|---|---|---|---|---|---|---|
| | **90° Dry Products** | | | **0°Wet Products** | | |
| | **No. pellets** | **Pellet(s) Signal** | **Plaque Signal** | **No. pellets** | **Pellet(s) Signal** | **Plaque Signal** |
| Inventive Ex. 1 | 4-5 | 150 | 17000 | 2 | 170 | 27000 |
| Inventive Ex. 2 | 2 | 177 | 34000 | 1 | 160 | 270000 |
| Inventive Ex. 3 | 1 | 220 | 39800 | 1 | 270 | 27600 |
| Inventive Ex. 4 | 1 | 180 | 42000 | 1 | 330 | 270000 |
| Inventive Ex. 5 | 1 | 180 | 42000 | 1 | 730 | 28000 |
| Inventive Ex. 6 | 1 | 1280 | 54000 | 1 | 1700 | 24000 |

Preferred embodiments include:
1. A composite material, comprising: (A) a particle comprising: (i) a core comprising one or more magnetic materials and (ii) a shell comprising silicon dioxide and (B) a polymer component selected from the group consisting of polyolefin homopolymers, polyolefin interpolymers, and combinations thereof, wherein the polymer component is free of free radical initiator.
2. The composite material according to embodiment 1, wherein the particle (A) is present in an amount from 2 to 50% by weight based on the total weight of the composite material.
3. The composite material according to any one of the preceding embodiments wherein five pellets, on a dry basis, formed from the composite material provides a signal of at least 120 using a Goring Kerr DSP2 metal detector when the composite material comprises at least 5 wt% particle (A).
4. The composite material according to any one of the preceding embodiments wherein a plaque, on a dry basis, formed from the composite material provides a signal of at least 16000 using a Goring Kerr DSP2 metal detector when the composite material comprises at least 5 wt% particle (A).
5.. The composite material according to any one of the preceding embodiments, wherein the polymer component is present in an amount from 50 to 98% by weight based on the total weight of the composite material.
6. The composite material according to any one of the preceding embodiments, wherein the polymer component is selected from the group consisting of polyethylene homopolymer, polyethylene/α-olefin copolymers, polypropylene homopolymer, polypropylene/α-olefin copolymers, and combinations thereof.
7. The composite material according to any one of the preceding embodiments, wherein the core comprises an iron oxide.
8. The composite material according to any one of the preceding embodiments, wherein the core is from 50 to 90% by weight of the particle (A), and the shell is from 10 to 50% by weight of the particle (A).
9. The composite material according to any one of the preceding embodiments, wherein the particle (A) is surface-modified.
10. The composite material according to any one of the preceding embodiments, wherein the polymer component comprises at least one polyethylene selected from the group consisting of olefin block copolymers, polyolefin elastomers, hPP, rPP, high density polyethylene, and combinations thereof.
11. The composite material according to any one of the preceding embodiments, wherein a contaminant content of the shell is less than 0.01% by weight.
12. The composite material according to any one of the preceding embodiments, wherein the shell is from 2 to 500 nm thick.
13. The composite material according to any one of the preceding embodiments, wherein the core comprises Fe, Co, Ni, Fe₃O₄, gamma-Fe₂O₃, MgFe₂O₄, MnFe₂O₄, CoFe₂O₄, CoPt₃, FePt, or a combination thereof.
14. The composite material according to any one of the preceding embodiments, wherein a BET surface area of a plurality of the particles (A) is from 5 to 500 m²/g.
15. The composite material according to any one of the preceding embodiments, wherein an average diameter of a plurality of the particles (A) is from 5 to 100 nm.
16. A method for producing a composite material comprising the steps of: providing particles which comprise: (i) a core comprising one or more magnetic materials and (ii) a shell comprising silicon dioxide; providing a polymer component selected from the group consisting of polyolefin homopolymers, polyolefin interpolymers, or a combinations thereof, wherein the polymer component is free of free radical initiator, and forming a mixture of the particles and polymer component.
17. An article which comprises the composite material of any one of the preceding embodiments, wherein the article is selected from the group consisting of sheets, laminates, and films.
18. The article of embodiment 17 wherein the article has a thickness from 0.25 mm to 4 mm.
19. A conveyor belt which comprises the article of embodiment 17 as an external layer, as an internal layer(s) or as a combination of internal layer(s) and external layer.
20. A method of self - healing or self - welding a damaged conveyor belt comprising the steps of:
   providing a conveyor belt of embodiment 19; and
   applying radiation to the conveyor belt.

## Claims

1. A composite material, comprising: (A) a particle comprising: (i) a core comprising one or more magnetic materials and (ii) a shell comprising silicon dioxide and (B) a polymer component selected from the group consisting of polyolefin homopolymers, polyolefin interpolymers, and combinations thereof, wherein the polymer component is free of free radical initiator.

2. The composite material according to claim 1, wherein the particle (A) is present in an amount from 2 to 50% by weight based on the total weight of the composite material.

3. The composite material according to any one of the preceding claims wherein five pellets, on a dry basis, formed from the composite material provides a signal of at least 120 using a Goring Kerr DSP2 metal detector when the composite material comprises at least 5 wt% particle (A); or wherein a plaque, on a dry basis, formed from the composite material provides a signal of at least 16000 using a Goring Kerr DSP2 metal detector when the composite material comprises at least 5 wt% particle (A).

4. The composite material according to any one of the preceding claims, wherein the polymer component is present in an amount from 50 to 98% by weight based on the total weight of the composite material.

5. The composite material according to any one of the preceding claims, wherein the polymer component is selected from the group consisting of polyethylene homopolymer, polyethylene/α-olefin copolymers, polypropylene homopolymer, polypropylene/α-olefin copolymers, and combinations thereof.

6. The composite material according to any one of the preceding claims, wherein the core comprises an iron oxide.

7. The composite material according to any one of the preceding claims, wherein the core is from 50 to 90% by weight of the particle (A), and the shell is from 10 to 50% by weight of the particle (A).

8. The composite material according to any one of the preceding claims, wherein the particle (A) is surface-modified.

9. The composite material according to any one of the preceding claims, wherein the polymer component comprises at least one polyethylene selected from the group consisting of olefin block copolymers, polyolefin elastomers, hPP, rPP, high density polyethylene, and combinations thereof.

10. The composite material according to any one of the preceding claims, wherein a contaminant content of the shell is less than 0.01% by weight.

11. The composite material according to any one of the preceding claims, wherein the shell is from 2 to 500 nm thick.

12. The composite material according to any one of the preceding claims, wherein the core comprises Fe, Co, Ni, Fe₃O₄, gamma-Fe₂O₃, MgFe₂O₄, MnFe₂O₄, CoFe₂O₄, CoPt₃, FePt, or a combination thereof.

13. The composite material according to any one of the preceding claims, wherein a BET surface area of a plurality of the particles (A) is from 5 to 500 m²/g.

14. The composite material according to any one of the preceding claims, wherein an average diameter of a plurality of the particles (A) is from 5 to 100 nm.

15. An article which comprises the composite material of any one of the preceding claims, wherein the article is selected from the group consisting of sheets, laminates, and films, preferably wherein the article has a thickness from 0.25 mm to 4 mm.
